(19) **Europäisches Patentamt / European Patent Office / Office européen des brevets**

(11) **EP 1 762 206 B1**

(12) **EUROPEAN PATENT SPECIFICATION**

(45) Date of publication and mention
of the grant of the patent:
**20.02.2013 Bulletin 2013/08**

(51) Int Cl.:
***A61F 13/02*** *(2006.01)*     *A61F 13/00* *(2006.01)*

(21) Application number: **05743783.2**

(22) Date of filing: **26.05.2005**

(86) International application number:
**PCT/JP2005/009628**

(87) International publication number:
**WO 2005/117782 (15.12.2005 Gazette 2005/50)**

(54) **PRESSURE-SENSITIVE ADHESIVE COVERING MATERIAL FOR APPLICATION TO SKIN**

BESCHICHTUNGSMATERIAL MIT KONTAKTKLEBER ZUM AUFBRINGEN AUF HAUT

MATÉRIAU DE COUVERTURE ADHÉSIF SENSIBLE À LA PRESSION POUR APPLICATION A LA PEAU

(84) Designated Contracting States:
**DE FR GB IT NL SE**

(30) Priority: **03.06.2004 JP 2004165257**

(43) Date of publication of application:
**14.03.2007 Bulletin 2007/11**

(73) Proprietor: **Alcare Co., Ltd.**
**Tokyo 1310046 (JP)**

(72) Inventor: **KUBO, Takabumi**
**Tokyo 1310046 (JP)**

(74) Representative: **Epping - Hermann - Fischer**
**Patentanwaltsgesellschaft mbH**
**Ridlerstrasse 55**
**80339 München (DE)**

(56) References cited:
**JP-A- 3 193 047    JP-A- 7 088 130**
**US-A- 4 846 164**

**Description**

Technical Field

The present invention relates to a pressure-sensitive adhesive covering material for application to the skin. More particularly, it relates to a pressure-sensitive adhesive covering material for application to the skin, comprising a laminated base material comprising a specific knitted fabric on one side of a specific film and a pressure-sensitive adhesive layer formed on the film on the side opposite to the side where the knitted fabric is laminated. The pressure-sensitive adhesive covering material for application to the skin of the present invention is particularly suitable for alleviating friction against the skin from the outside and preventing and treating pressure ulcers.

Background Art

When a person is confined to a bed because of a surgical operation or any disease or is forced to stay in a wheelchair or the like in an immobile state for a long time, pressure is locally concentrated particularly on a region where a bone projects. At this time, a compressive stress, a shear stress, a tensile stress or the like is applied to the inner body tissue, and a pressure ulcer is likely to occur.
In Patent document 1, a thin film pressure-sensitive adhesive bandage for preventing and treating a friction vesicle on the skin, comprising an embossed elastic film having one surface thereof coated with the pressure-sensitive adhesive is disclosed. In this pressure-sensitive adhesive bandage, the effective contact area of the film surface is reduced to 50% or less by embossing the film surface, and a friction coefficient of the surface is decreased.
However, according to the studies of the present inventor, it was found that, when this pressure-sensitive adhesive bandage is used for preventing and/or treating pressure ulcers, the embossed portion is easily deformed due to the elasticity of the film because a load is applied thereto from the human body, whereby the effective contact area is increased, which results in the increase of the friction force. As a result, the pressure-sensitive adhesive bandage is peeled off from the applied part or the pressure-sensitive adhesive bandage gets wrinkled, and a problem arises that a pressure ulcer occurs there on the contrary.
Further, in Patent document 2, a pressure-sensitive adhesive tape for medical use comprising a pressure-sensitive adhesive layer formed on one side of a non-woven fabric, in which the static friction coefficient of the surface layer of the non-woven fabric is in a specific range, is disclosed. It is described that this pressure-sensitive adhesive tape for medical use can be used as a surgical dressing tape for protecting an affected part or for covering, stitching and fixing a part undergoing surgery, a pressure-sensitive adhesive dressing tape for closure, a surgical pressure-sensitive adhesive tape for a surgical support material, a first-aid adhesive tape with a pad portion subjected to a special treatment, or the like. In this pressure-sensitive adhesive tape for medical use, a smoothing treatment of the non-woven fabric as a substrate is necessary in order to obtain a desired static friction coefficient, and the smoothing treatment is carried out by embossing the non-woven fabric or applying an inorganic filler or a pigment to the non-woven fabric. However, the stretchability or elasticity of the non-woven fabric is lowered by the embossment, therefore, when the non-woven fabric is applied to the skin, it cannot follow the skin and imparts an uncomfortable feeling during the application thereof. Further, the inorganic filler or pigment applied to the non-woven fabric is gradually exfoliated during use, therefore, the friction coefficient is gradually increased. As a result, in the same manner as the pressure-sensitive adhesive bandage in Patent document 1, the tape is peeled off from the applied part or the tape gets wrinkled, and a problem arises that a pressure ulcer occurs there on the contrary.
As described above, a conventional pressure-sensitive adhesive bandage or pressure-sensitive adhesive tape for medical use has problems that it is easily peeled off from the applied part or it easily gets wrinkled to cause the occurrence of a pressure ulcer on the contrary, and the like.
Patent Document 1: JP-A-3-178664

Patent Document 2: JP-A-11-9623

Disclosure of the invention

Problems that the Invention is to Solve

Accordingly, an object of the present invention is to provide a pressure-sensitive adhesive covering material for application to the skin that is hardly peeled off from the applied part, is less likely to get wrinkled and is suitable for particularly preventing and/or treating pressure ulcers.

Means for Solving the Problems

The present inventor made intensive studies in order to achieve the above object, and as a result, the inventor focused attention on a friction force applied to a surface of a living body as a cause of the occurrence of a pressure ulcer, and assumed that the reduction of this friction force would prevent the progression of a pressure ulcer and also lead to the prevention of the occurrence of a pressure ulcer. The inventor further advanced the studies based on this assumption, and has found that it is only necessary to form a pressure-sensitive adhesive layer.on the surface of a film having a specific elongation constituting a laminated base material together with a knitted fabric formed of filaments having a specific elongation. The inventor further has advanced the studies based on this finding and has completed the invention.

According to the invention, a pressure-sensitive adhesive covering material for application to the skin, characterized in that it comprises a laminated base material comprising a knitted fabric laminated on one side of a film and a pressure-sensitive adhesive layer formed on the film on the side opposite to the side where the knitted fabric is laminated, that the film has an elongation of 150 to 1,500% and that the knitted fabric is formed of filaments having an elongation of 1 to 200% is provided.

In the pressure-sensitive adhesive covering material for application to the skin of the invention, the filament forming the knitted fabric is a monofilament.

Further, in the pressure-sensitive adhesive covering material for application to the skin of the invention, it is preferred that the average diameter of the filament forming the knitted fabric is in the range of 15 to 200 $\mu$m.

Further, in the pressure-sensitive adhesive covering material for application to the skin of the invention, it is preferred that the knitted fabric has an elongation of 20 to 600%, that the unit weight of the knitted fabric is in the range of 10 to 100 g/m$^2$. The coverage of the film surface with the knitted fabric is in the range of 5 to 80%.

In the pressure-sensitive adhesive covering material for application to the skin of the invention, it is preferred that the ratio of elongations in the lengthwise direction and widthwise direction of the knitted fabric is in the range of 1.0 to 15.0.

In the pressure-sensitive adhesive covering material for application to the skin of the invention, it is preferred that the thickness of the film is in the range of 5 to 40 $\mu$m.

Further, in the pressure-sensitive adhesive covering material for application to the skin of the invention, it is preferred that the pressure-sensitive adhesive comprises a thermoplastic elastomer.

Further, in the pressure-sensitive adhesive covering material for application to the skin of the invention, it is preferred that the pressure-sensitive adhesive comprises a liquid component and/or a hydrophilic substance.

The pressure-sensitive adhesive covering material for application to the skin of the invention preferably has an elongation of 20 to 600% and a tensile stress at 20% elongation of 2.0 to 15.0 N/19 mm.

Further, the pressure-sensitive adhesive covering material for application to the skin of the invention preferably has a visible light transmittance of 2.5% or more.

In the pressure-sensitive adhesive covering material for application to the skin of the invention, the surface on the side of the knitted fabric preferably has a dynamic friction force of 3.0 N/40 cm$^2$ or less.

Further, the pressure-sensitive adhesive covering material for application to the skin of the invention is suitable for use as a pressure-sensitive adhesive covering material for preventing and/or treating pressure ulcers for the purpose of preventing the progression of a pressure ulcer by applying it to a part where the pressure ulcer has occurred and also preventing in advance the occurrence of a pressure ulcer by applying it to a part where a pressure ulcer may occur.

Advantage of the Invention

Because the pressure-sensitive adhesive covering material for application to the skin of the invention has an outer surface excellent in smoothness, it is excellent in alleviating the impact against the skin from the outside or the friction with a matter coming into contact with the covering material, is hardly peeled from the applied part and is less likely to get wrinkled. Therefore, it is suitable as a pressure-sensitive adhesive covering material for application to the skin. The pressure-sensitive adhesive covering material for application to the skin of the invention is suitable particularly for preventing and/or treating pressure ulcers. However, further by appropriately selecting a film, a knitted fabric and a pressure-sensitive adhesive which constitute the pressure-sensitive adhesive covering material for application to the skin, the applied part can be observed from the outside to permit effective performance of the prevention and/or treatment of pressure ulcers.

Best Mode for Carrying Out the Invention

A pressure-sensitive adhesive covering material for application to the skin (hereinafter referred to as merely a "pressure-sensitive adhesive covering material" in some cases) of the invention comprises a laminated base material comprising a knitted fabric laminated on one side of a film and a pressure-sensitive adhesive layer formed on the film on the side opposite to the side where the knitted fabric is laminated.

In the invention, it is necessary that the film constituting the laminated base material has an elongation of 150 to 1,500%. When the elongation is less than the above-mentioned lower limit, an uncomfortable feeling is caused during the application thereof because the film is difficult to follow the elongation of the skin, and the alleviating ability thereof against a shear stress from the outside or the skin is deteriorated, in a pressure-sensitive adhesive covering material for application to the skin obtained by using this film. Further, when the elongation is more than the above-mentioned upper limit, a wrinkle or a twist is liable to occur in the film during use, and smoothness is lowered.

The elongation of the film is preferably in the range of 160 to 500%.

A material constituting the film is not particularly limited as long as it has the above-mentioned elongation. Examples thereof include polyurethane; polyolefins such as polyethylene and polypropylene; olefin copolymers such as an ethylene-vinyl acetate copolymer (EVA), an ethylene-ethyl acrylate copolymer (EEA), an ethylene-methyl acrylate copolymer (EMA), an ethylene-methyl methacrylate copolymer (EMMA) and an ethylene-methacrylate polymer (EMAA); polyamides such as nylon 6 and nylon 66; polyesters such as polyethylene terephthalate and polybutylene terephthalate; polyvinyl alcohols; fluorocarbon resins; polyvinyl chloride; silicone; and the like. Among them, polyurethane, polyolefins, olefin copolymers and polyamides are preferred.

These materials may be used alone or as a mixture of two or more.

The thickness of the film may be preferably in the range of 5 to 40 $\mu$m, more preferably in the range of 10 to 30 $\mu$m. When the film is too thin, the pressure-sensitive adhesive covering material has not sufficient strength and may be torn by repetitive friction during use. On the other hand, when the film is too thick, the pressure-sensitive adhesive covering material becomes bulky. Therefore, when it is applied to the skin, an uncomfortable feeling is caused, and in particular, there is a fear that the adaptability thereof to a curving part is deteriorated.

The laminated base material to be used in the invention is formed by laminating a knitted fabric on the film. Here, the knitted fabric refers to a fabric composed of continuous stitches. The knitted fabric to be used in the invention is a knitted fabric comprised of filaments.

The filament is a monofilament. A smooth surface of a knitted fabric is easily obtained by using monofilaments, and a sheet or the like which comes into contact with the filaments hardly slips into the surface of the filaments, therefore, the knitted fabric comprised of the monofilaments has a good smoothness. Further, when a monofilament is used, diffuse reflection of light caused by a fiber can be suppressed, therefore, a see-through property is further enhanced.

Incidentally, the monofilament refers to a single filament (a continuous long fiber) which can be used alone, and the multifilament refers to a filament obtained by bundling two or more filaments.

It is necessary that the filament to be used in the invention has an elongation of 1 to 200%. The elongation thereof is preferably in the range of 1 to 40%. In case where the elongation of the filament is less than 1%, the entire knitted fabric formed of the filaments becomes stiff, and a pressure-sensitive adhesive covering material obtained by using this knitted fabric gives undesirably an uncomfortable feeling when it is applied to the skin. On the other hand, when the elongation thereof is more than 200%, a sheet or a cover for a mattress or a cushion or a clothing for the body such as an underwear (hereinafter, these are collectively referred to as simply "a sheet or the like" in some cases), which comes into contact with the knitted fabric, is easy to slip into the surface of the individual filaments constituting the knitted fabric, and as a result, a contact area with a sheet or the like is increased, therefore, the smoothness is lowered.

The filament has an average diameter of preferably 15 to 200 $\mu$m, more preferably 45 to 150 $\mu$m. When the average diameter thereof is too small, the control of the production step of the knitted fabric is troublesome, and there is a fear that the filament constituting the knitted fabric is easily cut by the friction with a sheet or the like during the use of the pressure-sensitive adhesive covering material. Further, when the average diameter thereof is too large, a level difference is generated between the knitted fabric and the film, which constitute the laminated base material. When a fiber product having an uneven surface, for example, comes into contact with the surface of this knitted fabric, the fiber product easily gets caught on this level difference, therefore there is a fear that the smoothness is lowered. Further, there is a fear that such a level difference causes roughness on the surface to undesirably degrade the touch of the surface to the skin.

Incidentally, with regard to the multifilament, the elongation and the average diameter of the filament described above represent, not the numerical value of each of the filaments constituting the multifilament, but the numerical value as the multifilament.

A material for the filament is not particularly limited.-Examples thereof include polyesters such as polyethylene terephthalate, polytrimethylene terephthalate and polybutylene terephthalate; polyolefins such as polyethylene and polypropylene; polyamides such as nylon 6 and nylon 66; acrylates, polyvinyl alcohols; polyvinyl chloride, polyvinylidene chloride; cellulose fibers such as viscose rayon, polynosic rayon, cuprammonium rayon, Lyocell and cellulose acetates (acetate, triacetate and the like); and the like.

Among them, polyesters and polyolefins are particularly preferred.

These materials may be used alone or as a mixture of two or more.

The knitted fabric to be used in the invention has an elongation in the lengthwise direction of preferably 20 to 600%, more preferably 40 to 600%. Further, it has an elongation in the widthwise direction of preferably 20 to 600%, more preferably 40 to 600%. Here, the lengthwise direction refers to a direction parallel to a direction of producing the knitted fabric and generally corresponds to a wale direction of the knitted fabric. On the other hand, the widthwise direction refers to a direction orthogonal to the lengthwise direction and generally corresponds to a course direction of the knitted fabric.

When the elongation of the knitted fabric is too small, the knitted fabric is difficult to follow the elongation of the skin and an uncomfortable feeling is caused during the application thereof, and also there is a fear that the alleviating ability thereof against a shear stress from the outside is deteriorated. On the other hand, when the elongation the knitted fabric is too large, the supporting force of the knitted fabric is lowered, and the structure of the stitches of the knitted fabric is liable to be deformed before the slide occurs on the interface between the surface of the knitted fabric and a sheet or the like coming into contact with the surface, therefore, there is a possibility of degrading the smoothness.

The ratio of elongations in the lengthwise and widthwise directions of this knitted fabric (in the case where the two elongations are different, the smaller elongation is defined as the denominator) is preferably in the range of 1.0 to 15.0, more preferably in the range of 1.0 to 8.0. When the elongation of either of the lengthwise and widthwise directions is too large, the surface of the knitted fabric easily gets wrinkled, and there is a fear that the smoothness is lowered.

In the invention, the use of the knitted fabric is essential. By the deformation of the structure of the loop of the knitted fabric, the elongation of the knitted fabric can fall within the above-mentioned range. It is difficult to achieve the above-mentioned elongation with the use of a fabric other than the knitted fabric such as a woven fabric or a non-woven fabric.

In the invention, a known structure can be adopted as the stitch structure of the knitted fabric. Specific examples include plain stitch, rib stitch, pearl stitch, smooth stitch and the like as weft knit, and denbigh stitch, cord stitch, atlas stitch, chain stitch, milanese stitch and the like as warp knit. In particular, in order to achieve a favorable elongation, it is preferred to adopt a weft knit structure.

The unit weight of the knitted fabric is preferably in the range of 10 to 100 $g/m^2$, more preferably in the range of 15 to 30 $g/m^2$. When the unit weight thereof is too small, there is a fear that strength sufficient to endure repetitive friction with a sheet or the like cannot be obtained. On the other hand, when the unit weight thereof is too large, the knitted fabric is bulky and causes an uncomfortable feeling when it is applied to the skin, and in particular, there is a fear that the adaptability to a curving part is lowered.

In the invention, it is necessary to use a laminated base material composed of a knitted fabric formed of filaments having an elongation falling within the above-mentioned specific range and a film having an elongation falling within the above-mentioned specific range. Because the knitted fabric supports the film, it is easy to handle the pressure-sensitive adhesive covering material when it is applied to the skin. Deformation or a wrinkle on the film hardly occurs during the application due to the elongations of the filament and the film falling within the above-mentioned ranges.

For the lamination of the knitted fabric and the film, a conventionally known method, for example, thermal fusion or bonding with an adhesive can be utilized, and the bonding strength between the knitted fabric and the film can be regulated by controlling pressure conditions, temperature conditions or the like.

The coverage of the film surface with the knitted fabric is in the range of 5 to 80%, preferably in the range of 10 to 40%. Here, the coverage with the knitted fabric refers to a percentage of the film area covered with the filaments constituting the knitted fabric to the film area constituting the laminated base material when the laminated base material is observed from just above the knitted fabric side. When the coverage with the knitted fabric is too small, the strength of the knitted fabric is lowered, and the filament may be cut by repetitive friction. Moreover, the contact area with a sheet or the like becomes too small, and the film under the knitted fabric becomes the contact face with a sheet or the like, therefore, there is a fear that the smoothness is lowered on the contrary. On the other hand, when the coverage with the knitted fabric is too large, the contact area with a sheet or the like becomes too large, therefore, there is a fear that the smoothness is lowered.

Further, by allowing the coverage with the knitted fabric to fall within the above-mentioned range, an advantage can be obtained that the gloss of the film surface can be suppressed, whereby when the pressure-sensitive adhesive covering material is applied to the skin, the covering material is hardly recognized.

The pressure-sensitive adhesive covering material of the invention comprises a pressure-sensitive adhesive layer formed on the film constituting the above-mentioned laminated base material on the side opposite to the side where the knitted fabric is laminated.

The pressure-sensitive adhesive is not particularly limited. Examples thereof include acrylic pressure-sensitive adhesives, rubber pressure-sensitive adhesives, vinyl ether pressure-sensitive adhesives, silicone pressure-sensitive adhesives and the like.

Examples of the acrylic pressure-sensitive adhesives include alkyl (meth)acrylates with an alkyl group having 4 to 18 carbon atoms, copolymers thereof and a pressure-sensitive adhesive resin which is a copolymer of any of the above alkyl (meth)acrylates with a functional monomer. Examples of the alkyl (meth)acrylates include butyl acrylate, isononyl acrylate, 2-ethylhexyl acrylate and the like. Examples of the functional monomer include (meth)acrylic acid, hydroxyalkyl (meth)acrylate, monoalkyl maleate, N-alkylmono-(meth)acrylamide, N,N-dimethylaminoalkyl (meth)acrylate and the like.

Examples of the rubber pressure-sensitive adhesives include pressure-sensitive adhesive compositions comprising a conjugated diene polymer, such as natural rubber, a polyisoprene rubber or a polybutadiene rubber, or any of various block copolymers such as an aromatic vinyl compound-conjugated diene block copolymer, and a tackifier blended therewith.

Specific examples of the block copolymers include a styrene-isoprene-styrene block copolymer (SIS), a styrene-butadiene-styrene block copolymer (SBS), a styrene-ethylene/propylene-styrene block copolymer (SEPS), a styrene-ethylene/butylene-styrene block copolymer (SEBS), a styrene-ethylene/ethylene/propylene-styrene block copolymer (SEEPS), a hydrogenated styrene-butadiene rubber (HSBR), a styrene-ethylene/butylene-olefin crystal block copolymer (SEBC), an olefin crystal-ethylene/butylene olefin crystal block copolymer (CEBC) and the like.

Examples of the tackifier include rosin resins, polyterpene resins, petroleum resins, terpenephenol resins and the like.

Examples of the vinyl ether pressure-sensitive adhesives include polyvinyl methyl ethers, polyvinyl ethyl ethers, polyvinyl isobutyl ethers and the like.

Examples of the silicone pressure-sensitive adhesives include polydimethyl siloxane, polydiphenyl siloxane and the like.

These pressure-sensitive adhesives may be used alone or in combination of two or more.

Among these pressure-sensitive adhesives, the rubber pressure-sensitive adhesives and the acrylic pressure-sensitive adhesives are preferred.

Further, it is preferred that the pressure-sensitive adhesive comprises a thermoplastic elastomer. Examples of the thermoplastic elastomers include the above-mentioned various block copolymers such as SIS, SBS, SEPS, SEBS, SEEPS, HSBR, SEBC and CEBC and, in addition, urethane (co)polymers and acrylic (co)polymers.

The addition of a liquid component to the above-mentioned pressure-sensitive adhesive enhances the adaptability to the skin, and imparts alleviating effect against stimulation or force from the outside.

As the liquid component to be added to the pressure-sensitive adhesive, a substance that is compatible with each component constituting the pressure-sensitive adhesive layer and is in a liquid state at normal temperature is preferred. Specific examples include liquid rubbers, polyhydric alcohols, synthetic oils, plant oils, animal oils and the like.

Examples of the liquid rubbers include a liquid isoprene rubber (LIR), a liquid styrene-isoprene rubber (LSIR), a liquid ethylene-propylene rubber (LEPR), a liquid styrene-ethylene-propylene rubber (LSEPR), a liquid polyisobutylene, a liquid polybutene and the like.

Examples of the polyhydric alcohols include polyethylene glycol, propylene glycol, dipropylene glycol, 1,3-butylene glycol, glycerol and the like.

Examples of the synthetic oils include higher fatty acids such as linolenic acid, linoleic acid, oleic acid and capric acid; higher fatty acid esters such as isopropyl myristate, octyldodecyl myristate, isononyl isononate, isopropyl palmitate and ethylhexyl palmitate; liquid paraffin, liquid isoparaffin; fatty acid triglycerides such as glycerol tri-2-ethyl hexanoate; silicone oils such as methyl polysiloxane, methylphenyl polysiloxane, dimethyl polysiloxane and aminosilicone and the like.

Examples of the plant oils include olive oil, olive squalane, macadamia nut oil, jojoba oil, squalane, almond oil, peanut oil, castor oil, coconut oil, palm oil, safflower oil, sunflower oil, cotton seed oil, hydrogenated coconut oil, hydrogenated palm oil, avocado oil, apricot kernel oil, grape seed oil and the like.

Examples of the animal oils include lanoline, turtle oil, beeswax, squalene, pristane and the like.

These liquid components may be used alone or in combination of two or more.

Among these liquid components, liquid rubbers are preferred, and in particular, a liquid isoprene rubber (LIR), a liquid styrene-isoprene rubber (LSIR), a liquid ethylene-propylene rubber (LEPR) and a liquid styrene-ethylene-propylene rubber (LSEPR) are preferred.

In particular, in the case where the pressure-sensitive adhesive is composed of a thermoplastic block copolymer elastomer, it is preferred to adopt a liquid rubber as the liquid component. Cohesiveness is thereby imparted to the pressure-sensitive adhesive layer, so that the pressure-sensitive adhesive layer becomes less likely to lose its shape and exhibits good flexibility to the skin.

The compounding amount of the liquid component is in the range of 4 to 80% by weight based on the total amount of the pressure-sensitive adhesive. When the compounding amount is in the range above, the liquid component can be added to the pressure-sensitive adhesive without losing the shape of the pressure-sensitive adhesive layer or causing cold flow, and good flexibility during the application thereof is exhibited.

The thickness of the pressure-sensitive adhesive layer is generally in the range of 15 to 5,000 $\mu$m, preferably in the range of 100 to 3,000 $\mu$m, more preferably in the range of 250 to 1,000 $\mu$m. Further, the thickness of the pressure-sensitive adhesive layer falling within this range realizes a sufficient adhesive force to the skin, and causes no uncomfortable feeling when the pressure-sensitive adhesive layer is applied to the skin. Further, the thickness of the pressure-sensitive adhesive layer falling within this range hardly causes deformation such as a wrinkle or unevenness in the pressure-sensitive adhesive layer and does not deteriorate the smoothness of the pressure-sensitive adhesive covering material.

When a hydrophilic substance is added to the pressure-sensitive adhesive to form a so-called hydrocolloid pressure-sensitive adhesive, sweat or an exudate from a wound is absorbed. Thus, in the case where the pressure-sensitive adhesive covering material is applied to the skin for a long time, a rash or an irritation or itching feeling caused by perspiration can be relieved. Here, the hydrophilic substance refers to a substance having a property of adsorbing or absorbing water.

The hydrophilic substance is not particularly limited. Preferred examples include pectin, sodium carboxymethyl cellulose (CMC-Na), guar gum, gelatin, karaya gum, alginic acid, chitin, chitosan, polyvinyl alcohol, polyacrylate, starch-acrylic acid graft polymers and the like.

In the invention, further a pharmaceutically active ingredient can be added to the pressure-sensitive adhesive for the purpose of a treatment of the skin, prevention of a wound, beautification or the like. For example, a substance having a moisturizing effect such as ceramide has an effect of restoring the skin damaged due to friction or exfoliation of the corneum toward a healthy skin, and the effect is effectively exhibited in conjunction with the cushioning effect of the pressure-sensitive adhesive covering material of the invention. Further, when γ-oryzanol having an effect of highly promoting blood flow or the like is added, vascular occlusion caused by body pressure is alleviated, and further effective prevention of a pressure ulcer can be expected.

Further, in the invention, an antioxidant, an agent for strengthening cohesiveness, a tackifier or the like can be added to the pressure-sensitive adhesive.

By forming the pressure-sensitive adhesive layer on the laminated base material comprising the film and the knitted fabric, the pressure-sensitive adhesive covering material for application to the skin of the invention can be obtained. As a method of providing the pressure-sensitive adhesive layer to the laminated base material, a conventionally known method can be used. For example, a pressure-sensitive adhesive is applied to a release paper and dried thereby to form a pressure-sensitive adhesive layer, then a laminated base material is laminated to the resulting pressure-sensitive adhesive layer, which is pressure-bonded by a pressure roller thereby to transfer the pressure-sensitive adhesive layer to the laminated base material, whereby the pressure-sensitive adhesive covering material can be obtained. As the form of the product of the pressure-sensitive adhesive covering material, a sheet form or a tape form can be used, and the size or the form may be appropriately selected in accordance with a part to be applied.

In the pressure-sensitive adhesive covering material for application to the skin of the invention, an elongation in the lengthwise direction (referring to the same direction as the lengthwise direction of the knitted fabric constituting the pressure-sensitive adhesive covering material) is preferably in the range of 20 to 600%, more preferably in the range of 40 to 600%. Further, an elongation in the widthwise direction (referring to the same direction as the widthwise direction of the knitted fabric constituting the pressure-sensitive adhesive covering material) is preferably in the range of 20 to 600%, more preferably in the range of 40 to 600%. When these elongations are not lower than the above lower limit, the covering material follows well the elongation of the skin, and an uncomfortable feeling is not caused during the application thereof, and also the alleviating ability thereof against a shear stress from the outside or the skin is favorably maintained. Further, when the elongations thereof are not more than the above upper limit, the pressure-sensitive adhesive covering material is not shifted from the applied position, deformation of the pressure-sensitive adhesive covering material is not caused, and it easily restore its original shape, therefore, the smoothness is favorably maintained.

In the pressure-sensitive adhesive covering material for application to the skin of the invention, the ratio of elongations in the lengthwise and widthwise directions (in the case where the two elongations are different, the smaller elongation is defined as the denominator) is preferably in the range of 1.0 to 15.0, more preferably in the range of 1.0 to 8.0. When the elongation of either of the lengthwise and widthwise directions is too large, the surface of the knitted fabric or the pressure-sensitive adhesive layer easily gets wrinkled, and there is a fear that the smoothness is lowered or an uncomfortable feeling is imparted during the application thereof.

In the pressure-sensitive adhesive covering material of the invention, a tensile stress at 20% elongation thereof is preferably in the range of 2.0 to 15.0 N/19 mm in the lengthwise direction and 2.0 to 15.0 N/19 mm in the widthwise direction, more preferably in the range of 2.0 to 11.0 N/19 mm in the lengthwise direction and 2.0 to 11.0 N/19 mm in the widthwise direction. When the tensile stress at 20% elongation is in the above range, the pressure-sensitive adhesive covering material for application to the skin of the invention follows well the elongation of the skin, and does not impart an uncomfortable feeling or a physical stimulation to the skin during the application thereof.

In the pressure-sensitive adhesive covering material for application to the skin of the invention having properties as described above, the surface on the side of the knitted fabric can have a dynamic friction force of 3.0 N/40 cm² or less, preferably 2.5 N/40 cm² or less. Because of the good smoothness thereof, stimulation to the skin caused by friction of the pressure-sensitive adhesive covering material is alleviated.

Further, in the pressure-sensitive adhesive covering material of the invention, a transparent film and a transparent pressure-sensitive adhesive layer can be adopted. In this case, the pressure-sensitive adhesive covering material excellent in a light transmission property can be obtained. When the pressure-sensitive adhesive covering material is applied, it exhibits an appearance similar to that of the skin, therefore, the aesthetic appearance during the application thereof is improved, and also the conditions of the skin can be observed for the purpose of preventing a pressure ulcer or treating a wound.

In the pressure-sensitive adhesive covering material for application to the skin of the invention, by combining the use of a polyurethane, a polyolefin, an olefin copolymer or a polyamide as the film and the use of an acrylic pressure-sensitive adhesive or a styrene block copolymer (SIS, SBS, SEPS, SEBS, SEEPS, HSBR, SEBC or the like) as the pressure-sensitive adhesive, the visible light transmittances of the knitted fabric, laminated base material and pressure-sensitive adhesive covering material can be made 20.0% or more, 5.0% or more and 2.5% or more, respectively. Thus, the aesthetic appearance when the pressure-sensitive adhesive covering material is applied to the skin is improved, and also the conditions of the skin can be easily observed while it is applied to the skin. Accordingly, because it is not necessary to peel the applied covering material when the conditions of the skin are to be observed, healing of pressure ulcers or the like can be promoted.

Examples

Hereinafter, the present invention will be described in more detail with reference to Examples. However, the invention is not limited to these. Incidentally, unless otherwise specified, % and part(s) are by weight.

Each property of the film, knitted fabric and pressure-sensitive adhesive covering material was measured in accordance with the following methods.

[Elongation of filament]

It is carried out in accordance with JIS L1013 (1999). First, a filament constituting a stitch is attached to the grips of a tensile testing machine (manufactured by Shimadzu Corporation, product name "Autograph AG-I") with a distance between grips of 50 mm in a slack state. Then, an elongation when applying a load which does not stretch the filament and makes the filament straight is read as a relaxation $E_0$ (mm), and an elongation $E_1$ (mm) at break is measured by stretching the filament at a tensile speed of 100 mm/min. The elongation of the filament is calculated with the following equation.

$$S_1 = \{ (E_1 - E_0) \ / \ (L + E_0) \} \ x \ 100$$

$S_1$: elongation of filament (%), $E_0$: relaxation (mm), $E_1$: elongation (mm) at break, L: distance between grips (= 50 mm)

[Elongation of knitted fabric]

It is carried out in accordance with JIS L1018 (1999). First, a specimen with a width of 25 mm and a length of 100 mm is attached to the grips of a tensile testing machine (manufactured by Shimadzu Corporation, product name "Autograph AG-I") with a distance between grips of 50 mm in a slack state. Then, a load that does not stretch a knitted fabric and makes the knitted fabric straight is applied, and the knitted fabric is stretched at a tensile speed of 100 mm/min, and an elongation $E_2$ (mm) at break is measured. The elongation of the knitted fabric is calculated with the following equation. The elongations are measured in both lengthwise and widthwise directions, respectively.

$$S_2 = (E_2 \ / \ L) \ x \ 100$$

$S_2$: elongation of knitted fabric (%), $E_2$: elongation (mm) at break, L: distance between grips (= 50 mm)

[Coverage with knitted fabric]

A view from just above the knitted fabric side of a laminated base material is photographed at a magnification of 50 times with a video microscope (manufactured by KEYENCE Co.). An area of the film coated with the stitches in the image observed at this time is measured and its percentage is calculated.

[Elongation of film]

A specimen with a width of 25 mm and a length of 100 mm is attached to the grips of a tensile testing machine (manufactured by Shimadzu Corporation, product name "Autograph AG-I") with a distance between grips of 50 mm.

Then, the specimen is stretched at a tensile speed of 100 mm/min, and an elongation $E_3$ (mm) at break is measured. The elongation of the specimen is calculated with the following equation.

$$S_3 = (E_3 / L) \times 100$$

$S_3$: elongation of film (%), $E_3$: elongation (mm) at break, L: distance between grips (= 50 mm)

[Elongation of pressure-sensitive adhesive covering material]

A specimen with a width of 19 mm and a length of about 100 mm is prepared and marks are made 50 mm apart from each other. This specimen is attached to the grips of a tensile testing machine (manufactured by Shimadzu Corporation, product name "Autograph AG-I") in such a manner that the region between the marks is placed between the grips. Then, the specimen is stretched at a tensile speed of 200 mm/min, and an elongation at break is calculated with the following equation. The elongations are measured in both lengthwise and widthwise directions, respectively.

$$elongation\ (\%) = \{(E - L) / L\} \times 100$$

S: elongation (%), E: length (mm) between marks at break, L: distance between marks (= 50 mm)

[Tensile stress at 20% elongation of pressure-sensitive adhesive covering material]

A specimen with a width of 19 mm and a length of about 100 mm is prepared and marks are made 50 mm apart from each other. This specimen is attached to the grips of a tensile testing machine (manufactured by Shimadzu Corporation, product name "Autograph AG-I") in such a manner that the region between the marks is placed between the grips. Then, the specimen is stretched at a tensile speed of 300 mm/min, and a tensile stress (N/19 mm) when the specimen is stretched by 20% is measured. The tensile stresses are measured in both lengthwise and widthwise directions, respectively.

[Dynamic friction force of pressure-sensitive adhesive covering material]

It is carried out in accordance with JIS K7125 (1999). As for a slide piece, unbleached muslin cotton fabric is used as a contact surface, and a square slide piece with a total mass of 100 g and a contact area of 40 $cm^2$ is used. A dynamic friction force (N/40 $cm^2$) is measured at a sliding speed of 300 mm/min. The dynamic friction forces are measured in both lengthwise and widthwise directions, respectively.

[Visible light transmittance]

A sample for measurement in which a 25 mm x 25 mm test piece is mounted on a transparent glass plate with a thickness of 0.13 to 0.17 mm is prepared. By using an ultraviolet-visible spectrophotometer (manufactured by Shimadzu Corporation, product name "UV-1650PC"), a light transmittance of this sample at a wavelength of 550 nm is measured and the measurement value is used as a visible light transmittance. The measurement is carried out for the respective test pieces of knitted fabric, laminated base material and pressure-sensitive adhesive covering material.

[Evaluation for application of pressure-sensitive adhesive covering material to skin (1): application feeling]

A pressure-sensitive adhesive covering material is cut into a piece with a width of 25 mm and a length of 50 mm and the piece is applied to a lower abdominal region of a test subject. Evaluation is carried out based on the three criteria (O: little irritation or an uncomfortable feeling, Δ: some irritation and uncomfortable feeling, ×: strong irritation and uncomfortable feeling) depending on a degree of irritation and an uncomfortable feeling that the test subject feels until 24 hours after it is applied.

[Evaluation for application of pressure-sensitive adhesive covering material to skin (2): condition after application]

Conditions of the pressure-sensitive adhesive covering material after the application for 24 hour are observed, and evaluation is carried out based on the three criteria (O: there is little occurrence, Δ: there is some occurrence, ×:

there is significant occurrence) depending on a degree of occurrence of deformation, wrinkle, peeling and separation of the pressure-sensitive adhesive covering material.

[Example 1]

15 parts of a styrene-isoprene-styrene block copolymer (SIS), 30 parts of a liquid isoprene (LIR) and 60 parts of a liquid styrene-isoprene copolymer rubber (LSIR) as liquid components, and 50 parts of sodium carboxycellulose (CMC-Na) as a hydrophilic substance were placed in a pressure kneader and mixed to homogeneity, whereby a pressure-sensitive adhesive A1 was prepared. This pressure-sensitive adhesive A1 was subjected to rolling with a pressure roller in such a manner that the thickness thereof becomes 600 $\mu$m, whereby a pressure-sensitive adhesive A1 layer was formed on a release paper.
Meanwhile, a smooth knitted fabric (elongation in the lengthwise direction: 60%, elongation in the widthwise direction: 300%) with a unit weight of 19.8 g/m$^2$ composed of a polyester monofilament with an average diameter of 52.5 $\mu$m and an elongation of 12% was bonded to a polyurethane film with a thickness of 17.5 $\mu$m and an elongation of 400% with a polyurethane pressure-sensitive adhesive in such a manner that the coverage with the knitted fabric becomes 21.1%, whereby a film/knitted fabric laminated base material S1 was obtained.
To the pressure-sensitive adhesive A1 layer, the laminated base material S1 was bonded on the film side, whereby a pressure-sensitive adhesive covering material C1 for application to the skin of the invention was obtained (Table 1).
This pressure-sensitive adhesive covering material C1 was evaluated for various properties such as an elongation and a tensile stress. The results are shown in Table 2.

[Example 2]

To a random copolymer obtained by copolymerizing 70 parts of butyl acrylate, 25 parts of 2-ethylhexyl acrylate, 3 parts of acrylic acid and 2 parts of methyl methacrylate, ethyl acetate was added as a solvent to give a pressure-sensitive adhesive A2 composed of a solution with a solid content of 50%. This pressure-sensitive adhesive A2 was applied to a release paper so that the thickness thereof becomes 50 $\mu$m, and then dried, whereby a pressure-sensitive adhesive A2 layer was formed on the release paper.
Meanwhile, a smooth knitted fabric (elongation in the lengthwise direction: 70%, elongation in the widthwise direction: 250%) with a unit weight of 30.0 g/m$^2$ composed of a polyamide monofilament with an average diameter of 63.7 $\mu$m and an elongation of 21% was bonded to a polyurethane film with a thickness of 20.0 $\mu$m and an elongation of 250% with a polyurethane pressure-sensitive adhesive so that the coverage with the knitted fabric becomes 33.1%, whereby a film/knitted fabric laminated base material S2 was obtained.
To the pressure-sensitive adhesive A2 layer, the laminated base material S2 was bonded on the film side, whereby a pressure-sensitive adhesive covering material C2 for application to the skin of the invention was obtained (Table 1).
This pressure-sensitive adhesive covering material C2 was evaluated for various properties such as an elongation and a tensile stress. The results are shown in Table 2.

[Example 3], comparative example

A pressure-sensitive adhesive covering material C3 (Table 1) was obtained in the same manner as in Example 2 except that the knitted fabric was changed to a smooth knitted fabric (elongation in the lengthwise direction: 44%, elongation in the widthwise direction: 80%) with a unit weight of 15.0 g/m$^2$ composed of a polyamide multifilament with an average diameter of 43.2 $\mu$m and an elongation of 19% and that the coverage of the film with the knitted fabric was changed to 19.3%. This pressure-sensitive adhesive covering material C3 was evaluated for various properties such as an elongation and a tensile stress. The results are shown in Table 2.

[Comparative example 1]

A pressure-sensitive adhesive covering material C4 (Table 1) was obtained in the same manner as in Example 1 except that the base material of the pressure-sensitive adhesive layer was changed so that it had a structure composed of only the film without using the knitted fabric. This pressure-sensitive adhesive covering material C4 was evaluated for various properties such as an elongation and a tensile stress. The results are shown in Table 2.

[Comparative example 2]

A pressure-sensitive adhesive covering material C5 (Table 1) was obtained in the same manner as in Example 2 except that the knitted fabric was changed to a plain woven fabric (elongation in the lengthwise direction: 32%, elongation in the widthwise direction: 32%) with a unit weight of 42.3 g/m$^2$ composed of a polyamide multifilament with an average diameter of 78.9 $\mu$m and an elongation of 32%, that the coverage of the film with the knitted fabric (woven fabric) was changed to 63.1%, and that the film was changed to a polyester film with a thickness of 20.0 $\mu$m and an elongation of 110%. This pressure-sensitive adhesive covering material C5 was evaluated for various properties such as an elongation and a tensile stress. The results are shown in Table 2.

[Comparative example 3]

A pressure-sensitive adhesive covering material C6 (Table 1) was obtained in the same manner as in Example 2 except that the knitted fabric was changed to a polyester spun lace non-woven fabric (elongation in the lengthwise direction: 31%, elongation in the widthwise direction: 38%) with a unit weight of 34.0 g/m$^2$ and that the coverage of the film with the non-woven fabric was changed to 32.3%. This pressure-sensitive adhesive covering material C6 was evaluated for various properties such as an elongation and a tensile stress. The results are shown in Table 2.
[Table 1]

| Pressure-sensitive adhesive covering material | | | C1 | C2 | C3 | C4 | C5 | C6 |
|---|---|---|---|---|---|---|---|---|
| Pressure-sensitive adhesive | | | | | | | | |
| | Symbol | | A1 | A2 | A3 | A4 | A5 | A6 |
| | SIS | | 15 | | | 15 | | |
| | LIR | | 30 | | | 30 | | |
| | LSIR | | 60 | | | 60 | | |
| | CMC-Na | | 50 | | | 50 | | |
| | Composition of monomer of acrylic pressure-sensitive adhesive (*1) | | | | | | | |
| | | BA | | 70 | 70 | | 70 | 70 |
| | | 2EHA | | 25 | 25 | | 25 | 25 |
| | | AA | | 3 | 3 | | 3 | 3 |
| | | MMA | | 2 | 2 | | 2 | 2 |
| Knitted fabric or the like | | | | | | | | |
| | Filament | | | | | | | |
| | | Material (*2) | PET | PAM | PAM | | PAM | PET |
| | | Form (*3) | Mono | Mono | Multi | | Multi | |
| | | Average diameter ($\mu$m) | 52.5 | 63.7 | 43.2 | | 78.9 | |
| | | Elongation (%): $S_1$ | 12 | 21 | 19 | | 32 | |
| | Fabric | | | | | | | |
| | | Fabric type | Knitted fabric | Knitted fabric | Knitted fabric | | Woven fabric | Non-woven fabric |
| | | Elongation: $S_2$ | | | | | | |
| | | Lengthwise direction (%) | 60 | 70 | 44 | | 32 | 31 |
| | | Widthwise direction (%) | 300 | 250 | 80 | | 32 | 38 |

(continued)

| Knitted fabric or the like | | | | | | | | |
|---|---|---|---|---|---|---|---|---|
| | | | Ratio of lengthwise / widthwise directions | 5.0 | 3.6 | 1.8 | | 1.0 | 1.2 |
| | | | Unit weight (g/m$^2$) | 19.8 | 30.0 | 15.0 | | 42.3 | 34.0 |
| | | | Visible light transmittance (%) | 63.2 | 60.3 | 30.0 | | 7.3 | 14.3 |
| Film | | | | | | | | |
| | | Material (*4) | | PU | PU | PU | PU | PET | PU |
| | | Thickness ($\mu$m) | | 17.5 | 20.0 | 20.0 | 17.5 | 20.0 | 20.0 |
| | | Elongation (%): S$_3$ | | 400 | 250 | 250 | 400 | 110 | 250 |
| Laminated base material | | | | | | | | |
| | | Symbol | | S1 | S2 | S3 | S4 | S5 | S6 |
| | | Coverage with knitted fabric (%) | | 21.1 | 33.1 | 19.3 | - | 63.1 | 32.3 |
| | | Visible light transmittance (%) | | 9.0 | 13.2 | 6.4 | 80.0 | 3.1 | 4.2 |
| Notes in Table 1<br>*1: composition (Part(s)) of monomer of acrylic pressure-sensitive adhesive<br>BA: butyl acrylate<br>2EHA: 2-ethylhexyl acrylate<br>AA: acrylic acid<br>MMA: methyl methacrylate<br>*2: material of filament<br>PET: polyester<br>PAM: polyamide<br>*3: form of filament<br>Mono: monofilament<br>Multi: multifilament<br>*4: material of film<br>PU: polyurethane<br>PET: polyester | | | | | | | | |

[Table 2]

| Physical properties for evaluation | | Example 1 | Example 2 | Example 3 | Comparative example 1 | Comparative example 2 | Comparative example 3 |
|---|---|---|---|---|---|---|---|
| Pressure-sensitive adhesive covering material | | C1 | C2 | C3 | C4 | C5 | C6 |
| Thickness of pressure-sensitive adhesive layer ($\mu$m) | | 600 | 50 | 50 | 600 | 50 | 50 |
| Elongation: S | | | | | | | |
| | Lengthwise direction (%) | 57 | 68 | 43 | 384 | 31 | 30 |

(continued)

| Elongation: S | | | | | | | |
|---|---|---|---|---|---|---|---|
| | Widthwise direction (%) | 297 | 245 | 80 | 382 | 31 | 36 |
| | Ratio of lengthwise / widthwise directions | 5.2 | 3.6 | 1.9 | 1.0 | 1.0 | 1.2 |
| Tensile stress at 20% elongation | | | | | | | |
| | Lengthwise direction (N/19 mm) | 10.0 | 8.5 | 14.8 | 2.4 | 51.0 | 21.0 |
| | Widthwise direction (N/19 mm) | 2.5 | 3.0 | 8.0 | 2.4 | 50.0 | 17.0 |
| Friction force | | | | | | | |
| | Lengthwise direction (N/40 cm$^2$) | 1.6 | 1.8 | 2.6 | 4.5 | 3.3 | 5.2 |
| | Widthwise direction (N/40 cm$^2$) | 1.6 | 1.8 | 2.8 | 4.4 | 3.5 | 5.2 |
| Visible light transmittance (%) | | 4.2 | 10.3 | 5.1 | 4.7 | 1.4 | 1.2 |
| Evaluation for adhering to skin | | | | | | | |
| | Application feeling | O | O | O | O | × | Δ |
| | Condition after application | O | O | O | × | × | × |

From the results of Table 2, the following conclusions can be drawn.

In comparative example 1, in which a base material of a pressure-sensitive adhesive covering material was formed only of a film without using a knitted fabric, although an uncomfortable feeling and the like occurred less during the application thereof, the film was deformed and got wrinkled, and an edge portion of the pressure-sensitive adhesive covering material was peeled or separated from the skin. It is considered that this is because the elongation of the film is increased due to not using a knitted fabric to increase the dynamic friction force thereof thereby lowering the smoothness.

In Comparative example 2 in which a laminated base material was formed using a film with a small elongation and a knitted fabric with a small elongation, although the dynamic friction force was smaller than that of Comparative example 1, irritation and an uncomfortable feeling during the application thereof were significant. It is considered that this is because the elongation of the pressure-sensitive adhesive covering material was small due to the small elongations of the film not enough to allow the knitted fabric and the covering material to follow the movement of the skin causing stimulation from the outside to be directly delivered to the applied part.

In Comparative example 3 in which a laminated base material was formed using a non-woven fabric instead of a knitted fabric, irritation and an uncomfortable feeling were caused during the application thereof. It is considered that this is because that the dynamic friction force of the pressure-sensitive adhesive covering material was increased due to the fuzzy surface of the non-woven fabric or the like, and that stimulation from the outside was directly delivered to the applied part without much alleviation on the surface of the non-woven fabric.

On the other hand, the pressure-sensitive adhesive covering materials of the invention gave a good feeling during the application thereof to the skin and caused no uncomfortable feeling. It is considered that this is because the pressure-sensitive adhesive covering materials showed a large elongation due to the structure of the knitted fabric to sufficiently follow the movement of the skin and that, as shown by the small dynamic friction force, less irritation was caused by contact with the pressure-sensitive adhesive covering material.

Further, it is understood that the visible light transmittance of the pressure-sensitive adhesive covering materials of the invention becomes high by appropriately selecting the film, the pressure-sensitive adhesive and the knitted fabric.

Further, it is understood that when a knitted fabric composed of monofilaments is used as the knitted fabric, the dynamic friction force is lower and the visible light transmittance is higher compared with the case of using multifilament.

## Claims

1.  A pressure-sensitive adhesive covering material for application to the skin, **characterized in that** it comprises a laminated base material comprising a knitted fabric laminated on one side of a film and a pressure-sensitive adhesive layer formed on the film on the side opposite to the side where the knitted fabric is laminated, that the film has an elongation of 150 to 1,500% that the knitted fabric is formed of monofilaments having an elongation of 1 to 200% and that the coverage of the film surface with the knitted fabric is in the range of 5 to 80%.

2.  The pressure-sensitive adhesive covering material for application to the skin according to claim 1, wherein the average diameter of the filament forming the knitted fabric is in the range of 15 to 200 $\mu$m.

3.  The pressure-sensitive adhesive covering material for application to the skin according to any one of the claims 1 or 2, wherein the knitted fabric has an elongation of 20 to 600%, the unit weight of the knitted fabric is in the range of 10 to 100 g/m$^2$.

4.  The pressure-sensitive adhesive covering material for application to the skin according to any one of the claims 1 to 3, wherein the ratio of elongations in the lengthwise and widthwise directions of the knitted fabric is in the range of 1.0 to 15.0.

5.  The pressure-sensitive adhesive covering material for application to the skin according to any one of the claims 1 to 4, wherein the thickness of the film is in the range of 5 to 40 $\mu$m.

6.  The pressure-sensitive adhesive covering material for application to the skin according to any one of the claims 1 to 5, wherein the pressure-sensitive adhesive comprises a thermoplastic elastomer.

7.  The pressure-sensitive adhesive covering material for application to the skin according to any one of the claims 1 to 6, wherein the pressure-sensitive adhesive comprises a liquid component and/or a hydrophilic substance.

8.  The pressure-sensitive adhesive covering material for application to the skin according to any one of the claims 1 to 7, which has an elongation of 20 to 600% and a tensile stress at 20% elongation of 2.0 to 15.0 N/19 mm.

9.  The pressure-sensitive adhesive covering material for application to the skin according to any one of the claims 1 to 8, which has a visible light transmittance of 2.5% or more.

10. The pressure-sensitive adhesive covering material for application to the skin according to any one of the claims 1 to 9, wherein the surface on the side of the knitted fabric has a dynamic friction force of 3.0 N/40 cm$^2$ or less.

11. The pressure-sensitive adhesive covering material for application to the skin according to any one of claims 1 to 10, which is for preventing and/or treating pressure ulcers.

## Patentansprüche

1.  Haftkleberabdeckmaterial zur Aufbringung auf die Haut, **dadurch gekennzeichnet, dass** es ein geschichtetes Basismaterial aufweist, das eine Maschenware, die auf eine Seite eines Films geschichtet ist, und eine Haftkleberschicht aufweist, die auf dem Film auf der Seite ausgebildet ist, die der Seite gegenüberliegt, wo die Maschenware geschichtet ist, dass der Film eine Dehnung von 150 bis 1500% aufweist, dass die Maschenware aus Monofilamenten gebildet wird, die eine Dehnung von 1 bis 200% aufweisen, und dass die Abdeckung der Filmoberfläche mit der Maschenware im Bereich von 5 bis 80% liegt.

2. Haftkleberabdeckmaterial zur Aufbringung auf die Haut nach Anspruch 1, wobei der durchschnittliche Durchmesser des Filaments, das die Maschenware bildet, im Bereich von 15 bis 200 $\mu$m liegt.

3. Haftkleberabdeckmaterial zur Aufbringung auf die Haut nach einem der Ansprüche 1 oder 2, wobei die Maschenware eine Dehnung von 20 bis 600% aufweist und das spezifische Gewicht der Maschenware im Bereich von 10 bis 100 g/m$^2$ liegt.

4. Haftkleberabdeckmaterial zur Aufbringung auf die Haut nach einem der Ansprüche 1 bis 3, wobei das Verhältnis der Dehnungen in die Längs- und Breitenrichtungen der Maschenware im Bereich von 1,0 bis 15,0 liegt.

5. Haftkleberabdeckmaterial zur Aufbringung auf die Haut nach einem der Ansprüche 1 bis 4, wobei die Dicke des Films im Bereich von 5 bis 40 $\mu$m liegt.

6. Haftkleberabdeckmaterial zur Aufbringung auf die Haut nach einem der Ansprüche 1 bis 5, wobei der Haftkleber ein thermoplastisches Elastomer aufweist.

7. Haftkleberabdeckmaterial zur Aufbringung auf die Haut nach einem der Ansprüche 1 bis 6, wobei der Haftkleber eine flüssige Komponente und/oder eine hydrophile Substanz aufweist.

8. Haftkleberabdeckmaterial zur Aufbringung auf die Haut nach einem der Ansprüche 1 bis 7, das eine Dehnung von 20 bis 600% und eine Zugspannung bei einer Dehnung von 20% von 2,0 bis 15,0 N/19 mm aufweist.

9. Haftkleberabdeckmaterial zur Aufbringung auf die Haut nach einem der Ansprüche 1 bis 8, das eine Durchlässigkeit für sichtbares Licht von 2,5% oder mehr aufweist.

10. Haftkleberabdeckmaterial zur Aufbringung auf die Haut nach einem der Ansprüche 1 bis 9, wobei die Oberfläche auf der Seite der Maschenware eine dynamische Reibungskraft von 3,0 N/40 cm$^2$ oder weniger aufweist.

11. Haftkleberabdeckmaterial zur Aufbringung auf die Haut nach einem der Ansprüche 1 bis 10, das zur Verhinderung und/oder zur Behandlung von Druckgeschwüren dient.

**Revendications**

1. Matériau de couverture adhésif sensible à la pression pour application à la peau, **caractérisé en ce qu'**il comprend un matériau de base stratifié comprenant un tissu tricoté appliqué par stratification sur un côté d'un film et une couche d'adhésif sensible à la pression formée sur le film sur le côté opposé au côté sur lequel le tissu tricoté est appliqué par stratification, **en ce que** le film a un allongement de 150 % à 1 500 %, **en ce que** le tissu tricoté est formé de monofilaments ayant un allongement de 1 % à 200 %, et **en ce que** le recouvrement de la surface du film par le tissu tricoté est de 5 % à 80 %.

2. Matériau de couverture adhésif sensible à la pression pour application à la peau selon la revendication 1, dans lequel le diamètre moyen du filament formant le tissu tricoté est de 15 $\mu$m à 200 $\mu$m.

3. Matériau de couverture adhésif sensible à la pression pour application à la peau selon la revendication 1 ou la revendication 2, dans lequel le tissu tricoté a un allongement de 20 % à 600 %, le poids unitaire du tissu tricoté étant de 10 g/m$^2$ à 100 g/m$^2$.

4. Matériau de couverture adhésif sensible à la pression pour application à la peau selon l'une quelconque des revendications 1 à 3, dans lequel le rapport des allongements dans le sens machine et dans le sens travers du tissu tricoté est de 1,0 à 15,0.

5. Matériau de couverture adhésif sensible à la pression pour application à la peau selon l'une quelconque des revendications 1 à 4, dans lequel l'épaisseur du film est de 5 $\mu$m à 40 $\mu$m.

6. Matériau de couverture adhésif sensible à la pression pour application à la peau selon l'une quelconque des revendications 1 à 5, dans lequel l'adhésif sensible à la pression comprend un élastomère thermoplastique.

7. Matériau de couverture adhésif sensible à la pression pour application à la peau selon l'une quelconque des revendications 1 à 6, dans lequel l'adhésif sensible à la pression comprend un constituant liquide et/ou une substance hydrophile.

8. Matériau de couverture adhésif sensible à la pression pour application à la peau selon l'une quelconque des revendications 1 à 7, ayant un allongement de 20 % à 600 % et une contrainte de traction de 2,0 N/19 mm à 15,0 N/19 mm à un allongement de 20 %.

9. Matériau de couverture adhésif sensible à la pression pour application à la peau selon l'une quelconque des revendications 1 à 8, ayant un facteur de transmission de la lumière visible de 2,5 % ou plus.

10. Matériau de couverture adhésif sensible à la pression pour application à la peau selon l'une quelconque des revendications 1 à 9, dans lequel la surface sur le côté du tissu tricoté a une force de frottement dynamique de 3,0 N/40 cm$^2$ ou moins.

11. Matériau de couverture adhésif sensible à la pression pour application à la peau selon l'une quelconque des revendications 1 à 10, destiné à la prévention et/ou au traitement d'escarres de décubitus.

**EP 1 762 206 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3178664 A
- JP 11009623 A